# EUROPEAN PATENT APPLICATION

(11) **EP 4 767 950 A1**
(43) Date of publication of application: **01.07.2026**
(21) Application number: 25227246.3
(22) Date of filing: 24.12.2025
(51) Int. Cl.: A61B 10/00, A61B 10/04

(54) **INGESTIBLE DEVICE AND PROCESS FOR COLLECTING GASTROINTESTINAL SAMPLES**

(30) Priority: 24.12.2024 IN 202411102448
(71) Applicant: Indian Institute of Technology Delhi, New Delhi 110016 (IN)
(72) Inventor: SRIVASTAVA, Sarvesh Kumar, 110016 Hauz Khas, New Delhi (IN); NEMA, Anshul, 110016 Hauz Khas, New Delhi (IN)
(74) Representative: Ponti & Partners, S.L.P

(57) **Abstract**

The present disclosure relates to a device for collecting gastrointestinal fluids/particles or samples. The present disclosure also relates to a process of collecting gastrointestinal fluids/particles or samples by the ingestible device. The ingestible device of the present disclosure is designed to non-invasively collect gastrointestinal particles, including biomolecules and components of the residing gut microbiome, with precise spatial resolution along the gastrointestinal (GI) tract. Upon deployment, the device's design and/or actuation mechanism facilitates the capture of gastrointestinal fluids and associated particles within those fluids. These samples are securely stored within the device until its natural egestion, after which the collected material can be subjected to further bio/chemical analyses.

## Description

### FIELD OF THE INVENTION

The present disclosure generally relates to the field of bio-medical engineering. The present disclosure relates to a device useful for collecting gastrointestinal fluids/particles or samples. More particularly, the present disclosure relates to an ingestible device for collecting gastrointestinal fluids/particles or samples. The present disclosure also relates to a process for collecting gastrointestinal fluids/particles or samples by the ingestible device.

### BACKGROUND OF THE INVENTION

The human gut microbiome or gastrointestinal (GI) microbiome governs key metabolic, protective and brain activity processes. However, non-invasive sampling of the gut microbiome has been a key bottleneck. Several efforts have been made in the past to overcome the drawbacks of the existing devices used for sampling.

Inés Garcia Viñado et al, April 30, 2024, a validation study to describe a non-invasive tool to collect small intestine content in post weaning pigs. It is a data-driven study in porcine model.

Jose Fernando Waimin et al, April 24, 2020, discloses a smart capsule for non-invasive sampling and studying of gastrointestinal microbiome. The capsule is biodegradable non-pH-sensitive material that cannot provide GI site-specificity.

Published US application US20210228157A1 discloses a non-invasive system for sampling gastrointestinal microbiota with their associated environment for discovery, characterization, medical research, diagnostics, and treatment using an ingestible, non-digestible sampling capsule. The capsule device, with ports open for acquiring a sample of gastrointestinal microbiota and content, is placed inside an immediate or delayed release capsule. When the outer capsule dissolves according to its specifications, enteric fluid and content enters the sampling capsule through the ports triggering a hydrophilic stop to release a pulling force to close the ports by enveloping the capsule outer casing over the inner casing, capturing the sample, and holding it sealed until it completes passage through the digestive track, and is recovered from feces for analysis.

Granted US patent US11766249B2 discloses devices and methods for collecting gastrointestinal samples using a capsule-shaped device that is swallowed. The patent discloses a device for collecting gastrointestinal samples. The device comprises a capsule; and a tube-shaped body wound, twisted or folded within said capsule, the body comprising an open end and a closed end.

None of the prior art documents provide any disclosure about an ingestible device and process for collecting gastrointestinal fluids/particles or samples that allows safe, precise and non-invasive isolation and collection of the GI microbiome, gastrointestinal fluids/particles, including biomolecules and components of the residing gut microbiome, with precise spatial resolution along the gastrointestinal (GI) tract.

### OBJECTIVE OF THE INVENTION

The primary objective of the present disclosure is to provide a device for collecting gastrointestinal fluids/particles or samples.

Another objective of the present disclosure is to provide an ingestible device for collecting gastrointestinal fluids/particles or samples.

Yet another objective of the present disclosure is to provide a process of collecting gastrointestinal fluids/particles or samples by the ingestible device.

Still another objective of the present disclosure is to provide an ingestible device and process for non-invasive collection of gastrointestinal fluids/particles by the ingestible device, including biomolecules and components of the residing gut microbiome, with precise spatial resolution along the gastrointestinal (GI) tract.

### SUMMARY OF THE INVENTION

The present invention provides an ingestible device designed to non-invasively collect gastrointestinal particles, including biomolecules and components of the residing gut microbiome, with precise spatial resolution along the gastrointestinal (GI) tract. The device is configured to capture samples along the entire length of the GI tract, including the stomach, pylorus, duodenal region, and intestinal lumen including ileum, jejunum, large intestine, and colon. Upon deployment, the device's design and/or actuation mechanism facilitates the capture of gastrointestinal fluids and associated particles within those fluids. These samples are securely stored within the device until its natural egestion, after which the collected material can be subjected to further bio/chemical analyses.

An ingestible device for collecting samples from a subject, the device comprising:
- a core shell (2) having a plurality of outer wall defining an inner volume;
- a separate passage (6) disposed in the inner volume and adapted to divide the inner volume into one or more chambers selected from activation chamber (3) and sample chamber (4);
- an inlet passage (8) in fluid communication with the chamber selected from activation chamber (3) and sample chamber (4) of the core shell;
- an activation mechanism (5,12) disposed in the chamber selected from activation chamber (3) and sample chamber (4) and is adapted to allow ingress of sample from the inlet passage (8) to the sample chamber (4) through the separator passage (6).

These and other features, aspects, and advantages of the present subject matter will be better understood with reference to the following description. This summary is provided to introduce a selection of concepts in a simplified form.

### BRIEF DESCRIPTION OF THE ACCOMPANYING DRAWINGS

The following figures form part of the present specification and are included to further illustrate aspects of the present disclosure. The disclosure may be better understood by reference to the figures in combination with the detailed description of the specific embodiments presented herein.

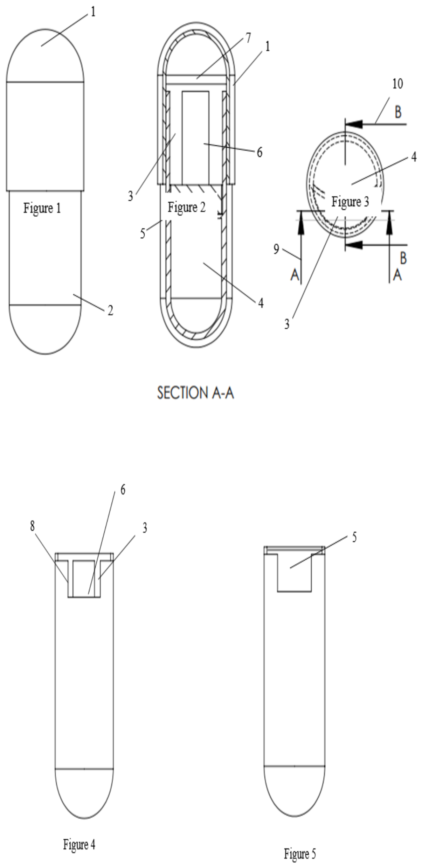

Figure 1 illustrates a perspective view of an embodiment of the device of the present disclosure, a device-1 before ingesting with shell.
Figure 2 illustrates a sectional view of a device-1 before ingesting with shell. Where section plane is front plan A-A.
Figure 3 illustrates a top view of a device-1 showing section plane A-A and B-B.
Figure 4 illustrates the front view of a device-1 after dissolving outer shell.
Figure 5 illustrates a front view of a device-1 after activation of pre-polymer mixture or swelling of the hydrogel.

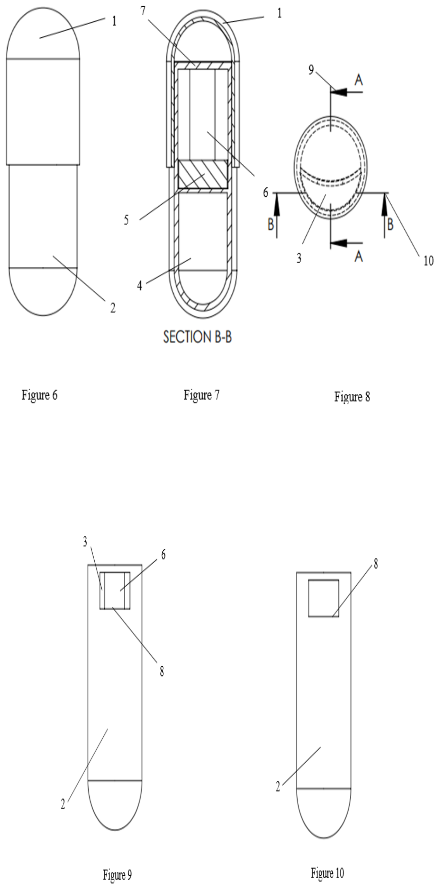

Figure 6 illustrates a perspective view of another embodiment of the present disclosure, a device-2 before ingesting with shell.
Figure 7 illustrates a sectional view of a device-2 before ingesting with shell. Where section plane is front plan B-B.
Figure 8 illustrates a top view of device-2 showing section plane A-A and B-B.
Figure 9 illustrates a front view of a device-2 after dissolving outer shell.
Figure 10 illustrates a front view of a device-2 after activation of pre-polymer mixture or swelling of the hydrogel.

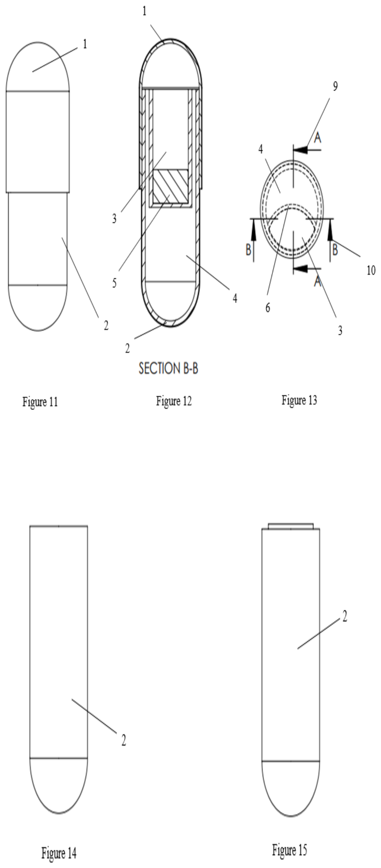

Figure 11 illustrates a perspective view of another embodiment of the present disclosure, a device-3 before ingesting with shell.
Figure 12 illustrates a sectional view of a device-3 before ingesting with shell. Where section plane is front plan B-B.
Figure 13 illustrates a top view of a device-3 showing section plane A-A and B-B.
Figure 14 illustrates a front view of a device-3 after dissolving outer shell.
Figure 15 illustrates a front view of a device-3 after activation of pre-polymer mixture or swelling of the hydrogel.

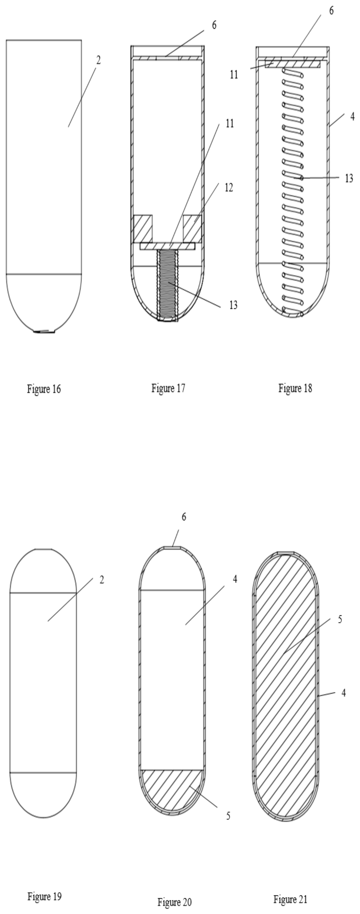

Figure 16 illustrates a perspective view of another embodiment of the present disclosure, device 4.
Figure 17 illustrates a front sectional view of device 4 before activation of spring.
Figure 18 illustrates a front sectional view of device 4 after activation of spring.
Figure 19 illustrates a perspective view of another embodiment of the present disclosure, device 5.
Figure 20 illustrates a front sectional view of device 5 before activation of pre-polymer mixture or hydrogel swelling.
Figure 21 illustrates a front sectional view of device 5 after activation of pre-polymer mixture or swelling of the hydrogel.
Figure 22 is a graphical representation of FTIR study of PEGDA 575 polymerization with citric acid, ascorbic acid, tartaric acid focusing on wave number covering: a) 500 to 4000 cm⁻¹, and b)1500 to 1800 cm⁻¹ region.
Figure 23 depicts results of the histological assessment of a cross-sectional area of the excised small intestine tissue (jejunum).
Figure 24 depicts excised murine tissues showing methylene blue marking at the site of ingestible microdevice activation: A) stomach region with visible blue staining; B) caecum region with the excised device.

### REFERENCE NUMERALS

1. It is outer shell made up of material which dissolves at a pH which is appropriate for sample collection.
2. Device outer shell use for sampling
3. It is small compartment used for chemical activation (e.g., pre-polymer or hydrogel filling, among others) or mechanical actuation (e.g., spring, coil, or retracting-band mechanisms, among others).
4. It is a sampling compartment used to collect samples for further testing.
5. It is a hydrogel or pre-polymer mixture used to close sample compartment opening in device 1 and device 2. And for tracking in device 3.
6. It is a separator passage which allows the sample to pass from pre-polymer mixture compartment or hydrogel swelling compartment to sampling compartment.
7. Outer walls used to cover sampling and hydrogel compartment in device 1 and used to cover only sampling compartment in device 2 and allow user to easily fill hydrogel or pre-polymer mixture in the compartment.
8. It is inlet passage provided on device wall to allow sample fluid to enter into the device.
9. Shows the section plane A-A.
10. Shows the section plane B-B.
11. It is the supporter plate to close the sampling compartment after sample collection.
12. It is the dissolvable block used to restrict the movement of supporter plate before sample collection.
13. It is spring act as an actuator to provide movement to supporter plate.

Further, skilled artisans will appreciate that elements in the drawings are illustrated for simplicity and may not have necessarily been drawn to scale. For example, the flow charts illustrate the method in terms of the most prominent steps involved to help to improve understanding of aspects of the present invention. Furthermore, in terms of the construction of the device, one or more components of the device may have been represented in the drawings by conventional symbols, and the drawings may show only those specific details that are pertinent to understanding the embodiments of the present invention so as not to obscure the drawings with details that will be readily apparent to those of ordinary skill in the art having benefit of the description herein.

### DETAILED DESCRIPTION OF THE INVENTION

Those skilled in the art will be aware that the present disclosure is subject to variations and modifications other than those specifically described. It is to be understood that the present disclosure includes all such variations and modifications. The disclosure also includes all such steps of the process, features of the product, referred to or indicated in this specification, individually or collectively, and any and all combinations of any or more of such steps or features.

### Definitions

For convenience, before further description of the present disclosure, certain terms employed in the specification, and examples are collected here. These definitions should be read in the light of the remainder of the disclosure and understood as by a person of skill in the art. The terms used herein have the meanings recognized and known to those of skill in the art, however, for convenience and completeness, particular terms and their meanings are set forth below.

The articles "a", "an" and "the" are used to refer to one or to more than one (i.e., to at least one) of the grammatical object of the article.

The term "some" as used herein is defined as "none, or one, or more than one, or all." Accordingly, the terms "none," "one," "more than one," "more than one, but not all" or "all" would all fall under the definition of "some." The term "some embodiments" may refer to no embodiments or to one embodiment or to several embodiments or to all embodiments. Accordingly, the term "some embodiments" is defined as meaning "no embodiment, or one embodiment, or more than one embodiment, or all embodiments."

The terms "comprise" and "comprising" are used in the inclusive, open sense, meaning that additional elements may be included. It is not intended to be construed as "consists of only".

Throughout this specification, unless the context requires otherwise the word "comprise", and variations such as "comprises" and "comprising", will be understood to imply the inclusion of a stated element or step or group of element or steps but not the exclusion of any other element or step or group of element or steps.

The term "including" is used to mean "including but not limited to". "Including" and "including but not limited to" are used interchangeably.

As used herein, the term "pre-polymer" means a compound or composition comprising a monomeric unit, an oligomeric unit, or a shorter polymeric unit, which may polymerise or further polymerise to form a polymer. Structurally, these moieties may comprise one or a plurality of chemical moieties which can polymerise. Such chemical moieties are for instance carbon-carbon double bonds. The terms "monomer", "monomeric units" or "monomeric subunits" as used herein refer to all of the monomeric units, oligomeric units, or shorter polymeric units, which may polymerise or further polymerise to form a polymer.

As used herein, the terms "absorb", "adsorb", "enmesh", "encapsulate", "trap" and "entrap" and other grammatical derived forms thereof, in connection with absorbing microbiome and other biomolecules into a hydrogel and/or polymer, are used synonymously herein.

As used herein, the terms "hydrogel" "swelling" "polymer swelling" "polymerization" "redox polymerization" "Free radical polymerization" and "in situ polymerization" are synonymous and refers to combination of one or more monomeric units and/or volumetric expansion of the material.

Unless defined otherwise, all technical and scientific terms used herein have the same meaning as commonly understood by one of ordinary skill in the art to which this disclosure belongs. Although any methods and materials similar or equivalent to those described herein can be used in the practice or testing of the disclosure, the preferred methods, and materials are now described. All publications mentioned herein are incorporated herein by reference.

The present disclosure is not to be limited in scope by the specific embodiments described herein, which are intended for the purposes of exemplification only. Functionally equivalent products and methods are clearly within the scope of the disclosure, as described herein.

The present disclosure relates to a device for collecting gastrointestinal fluids/particles or samples. The present disclosure particularly relates to an ingestible device for collecting gastrointestinal fluids/particles or samples. The present disclosure also relates to a process of collecting gastrointestinal fluids/particles or samples by the device. The present disclosure relates to ingestible devices and a process, that allows safe, precise and non-invasive isolation of the GI microbiome. The present disclosure relates to an ingestible device and methods thereof for non-invasive collection of gastrointestinal fluids/particles, including biomolecules and components of the residing gut microbiome, with precise spatial resolution along the gastrointestinal (GI) tract. The present disclosure provides a non-invasive collection of gastrointestinal fluids/particles, including biomolecules and components of the residing gut microbiome, with precise spatial resolution along the gastrointestinal (GI) tract. The present disclosure is useful for disease biomarker identification, microbiome research, and other applications requiring gastrointestinal sampling without invasive procedures.

In an aspect of the present disclosure, there is provided an ingestible device for collecting samples from a subject. The device of the present disclosure is adapted for deployment within a gastrointestinal (GI) tract, configured to perform concurrent collection of biological content and in-situ anatomical marking. The device of the present disclosure is utilized in any mammalian subject, including human and non-human animals, and is adaptable across species-specific anatomical and physiological variations. The device of the present disclosure allows safe, precise and non-invasive isolation and collection of the GI microbiome, gastrointestinal fluids/particles, including biomolecules and components of the residing gut microbiome, with precise spatial resolution along the gastrointestinal (GI) tract.

In an embodiment of the present disclosure, there is provided an ingestible device for collecting samples from a subject, the device comprising:
- a core shell (2) having a plurality of outer wall defining an inner volume;
- a separator passage (6) disposed in the inner volume and adapted to divide the inner volume into one or more chambers selected from activation chamber (3) and sample chamber (4);
- an inlet passage (8) in fluid communication with the chamber selected from activation chamber (3) and sample chamber (4) of the core shell;
- an activation mechanism (5, 12) disposed in the chamber selected from activation chamber (3) and sample chamber (4) and is adapted to allow ingress of sample from the inlet passage (8) to the sample chamber (4) through the separator passage (6).

At least a portion of the activation chamber (3) is coated or sealed with a pH-sensitive protective layer such as an enteric coating, or a combination thereof with animal or plant-based Gelatine with additives, that inhibits premature release or activation under acidic conditions (e.g., gastric pH ≈ 1-3) and dissolves or ruptures in more neutral or alkaline conditions (e.g., intestinal pH ≈ 5.5-7.5). Upon degradation of this coating, two critical events are triggered: Release of the GI lumen marking agent or payload delivery into the local GI environment, thereby allowing visual, optical, or spectroscopic detection of the location of sampling.

In an embodiment of the present disclosure, there is provided an ingestible device for collecting samples from a subject as described herein, wherein the device further comprises a pH sensitive protective outer shell (1) mounted partially on the outer surface of the core shell (2).

In an embodiment of the present disclosure, there is provided an ingestible device for collecting samples from a subject as described herein, wherein the pH sensitive protective outer shell (1) is selected from enteric coating and a combination of enteric coating and animal/plant-based gelatine with additives.

In an embodiment of the present disclosure, there is provided an ingestible device for collecting samples from a subject as described herein, wherein the device further comprises a top cover (7) disposed at a mouth of the core shell and covered by the pH sensitive protective outer shell (1).

In an embodiment of the present disclosure, there is provided an ingestible device for collecting samples from a subject as described herein, wherein the top cover (7) covers the sample chamber (4) and activation chamber (3).

In an embodiment of the present disclosure, there is provided an ingestible device for collecting samples from a subject as described herein, wherein the top cover (7) covers the sample chamber (4).

In an embodiment of the present disclosure, there is provided an ingestible device for collecting samples from a subject as described herein, wherein the top cover (7) allows users to easily fill the hydrogel or pre-polymer mixture in the chamber or compartment.

The device of the present disclosure further comprises a detectable marking agent chamber disposed above the activation chamber. The detectable marking agent chamber comprises detectable marking agent that includes, but is not limited to, one or more dyes, pigments, fiducial markers, radiopaque agents like BaSO₄, nanoparticles, enzymes coated particles, or chromophores or fluorophores or dyes, like azo dyes among others, capable of producing a persistent or semi-persistent colorimetric change upon contact with biological tissue. Suitable agents may include methylene blue, indocyanine green, toluidine blue, crystal violet, or their derivatives, or any biologically acceptable fiducial markers or contrast agents.

In an embodiment of the present disclosure, there is provided an ingestible device for collecting samples from a subject as described herein, wherein the device comprises a detectable marking agent chamber (3) disposed in the inner volume of core shell (1).

In an embodiment of the present disclosure, there is provided an ingestible device for collecting samples from a subject as described herein, wherein the device comprises a detectable marking agent chamber (3) covered partially by the pH sensitive protective outer shell (1).

In an embodiment of the present disclosure, there is provided an ingestible device for collecting samples from a subject as described herein, wherein the detectable marking agent is selected from the group consisting of dyes, pigments, fiducial markers, radiopaque agents, nanoparticles, enzymes coated particles, chromophores and fluorophores.

In an embodiment of the present disclosure, there is provided an ingestible device for collecting samples from a subject, the device comprising:
- a core shell (2) having a plurality of outer wall defining an inner volume;
- a pH sensitive protective outer shell (1) mounted partially on the outer surface of the core shell (2);
- a separate passage (6) disposed in the inner volume and adapted to divide the inner volume into one or more chambers selected from activation chamber (3) and sample chamber (4);
- a top cover (7) disposed at a mouth of the core shell and covered by the pH sensitive protective outer shell (1);
- a detectable marking agent chamber (3) disposed in the inner volume of core shell and is covered partially by the pH sensitive protective outer shell (1);
- an inlet passage (8) in fluid communication with the chamber selected from activation chamber (3) and sample chamber (4) of the core shell; and
- an activation mechanism (5) disposed in the chamber selected from activation chamber (3) and sample chamber (4) and is adapted to allow ingress of sample from the inlet passage (8) to the sample chamber (4) through the separator passage (6).

In an embodiment of the present disclosure, there is provided an ingestible device for collecting samples from a subject as described herein, wherein the activation mechanism is selected from the group consisting of chemical, electrical, and mechanical based system.

In an embodiment of the present disclosure, there is provided an ingestible device for collecting samples from a subject as described herein, wherein the activation mechanism is adapted to close the sample chamber upon the lapse of a predefined period of time from a first instance of collection of sample in the sample chamber.

Ingress of luminal fluid into the internal compartment or sample chamber (4), initiating an in-situ polymerization reaction that forms a hydrogel matrix entrapping luminal contents, such as microbial cells, genetic material, metabolites, or biomolecules. The polymerization reaction may occur under ambient GI conditions without external triggers and forms a solid or semi-solid matrix encapsulating a spatially defined biological sample.

In preferred configurations, the device geometry is selected from the group consisting of 3D printed, molded, or assembled using biocompatible materials.

Activation mechanism of the device of the present disclosure is autonomously governed by environmental triggers, such as pH, temperature, chemical or enzymatic activity. This system enables site-resolved sampling across multiple GI segments (e.g., stomach, small intestine, cecum, colon), and the marked locations facilitate post-retrieval anatomical correlation, endoscopic validation, or histopathological evaluation.

In an embodiment of the present disclosure, there is provided an ingestible device for collecting samples from a subject as described herein, wherein the activation mechanism is a chemical-based system.

In an embodiment of the present disclosure, there is provided an ingestible device for collecting samples from a subject as described herein, wherein the chemical-based activation mechanism is a chemical polymerizable formulation comprises at least one polymerizable monomer or hydrogel pre-polymer and a redox/radical initiator.

In an embodiment of the present disclosure, there is provided an ingestible device for collecting samples from a subject as described herein, wherein the hydrogel pre-polymer comprises a single type of pre-polymer compound. The corresponding hydrogel polymer which forms upon deployment of the device is a homopolymer.

In an embodiment of the present disclosure, there is provided an ingestible device for collecting samples from a subject as described herein, wherein the hydrogel pre-polymer is a composition comprising two or more pre-polymer compounds. The corresponding hydrogel polymer is a copolymer.

In an embodiment of the present disclosure, there is provided an ingestible device for collecting samples from a subject as described herein, wherein the hydrogel pre-polymer compound comprises olefinic (i.e. unsaturated) monomers which are water-soluble. Examples of such moieties are 2-acrylamido-2-methylpropanesulfonic acid (AMPS), acrylic acid, 3-sulphopropyl acrylate (SPA), and salts thereof. Particularly useful salts include, for example, the sodium, potassium, magnesium, calcium, lithium, and ammonium salts of the water-soluble monomers.

In an embodiment of the present disclosure, there is provided an ingestible device for collecting samples from a subject as described herein, wherein the monomer comprises a moiety selected from the group consisting of carboxylic acids, carboxylic acid anhydrides, sulfonic acids, dimethyl acrylamide, diacetone acrylamide, hydroxyl ethyl methacrylate, hydroxyl ethyl acrylate, dimethylaminoethyl acrylate, dimethylaminoethyl methacrylate, ethoxy ethoxy ethyl methacrylate, ethoxy ethyl methacrylate, methyl acrylate, ethyl acrylate, propyl acrylate, butyl acrylate, methyl methacrylate, acrylic acid, methacrylic acid, maleic acid, cinnamic acid, itaconic acid, crotonic acid, ethacrylic acid, citoconic acid, mesaconic acid, fumaric acid, β-sterylacrylic acid, acrylate esters, acrylamides, olefins, vinyl esters, vinyl ethers, vinyl amides, 2-acrylamido-2-methylpropane sulfonic acid (AMPS), and 3-sulphopropyl acrylate (SPA).

In an embodiment of the present disclosure, there is provided an ingestible device for collecting samples from a subject as described herein, wherein the polymerizable monomer of chemical polymerizable formulation is selected from the group consisting of a poly (ethylene glycol) derivative, acrylated poly (ethylene glycol) derivative, and methacrylated poly (ethylene glycol) derivative.

In an embodiment of the present disclosure, there is provided an ingestible device for collecting samples from a subject as described herein, wherein the monomer is a diacrylate monomer.

In an embodiment of the present disclosure, there is provided an ingestible device for collecting samples from a subject as described herein, wherein the monomer is poly (ethylene glycol) diacrylate (PEGDA).

In a specific embodiment, of the present disclosure, there is provided an ingestible device for collecting samples from a subject as described herein, wherein the PEGDA has an average molecular weight of 300 to 850 g/mol, such as 400 to 750 g/mol, such as about 575 g/mol, such as 575 g/mol.

In a specific embodiment, of the present disclosure, there is provided an ingestible device for collecting samples from a subject as described herein, wherein the hydrogel polymer comprises a crosslinked polymer. The cross-linked polymer may expand in volume upon exposure to fluids, such as aqueous medium.

In an embodiment of the present disclosure, there is provided an ingestible device for collecting samples from a subject as described herein, wherein the redox/radical initiator system of chemical polymerizable formulation is selected from transition metal salts such as iron (III) chloride, peroxides, persulfates, and organic reducing agents.

In an embodiment of the present disclosure, there is provided an ingestible device for collecting samples from a subject as described herein, wherein the organic reducing agents of redox/radical initiator system of chemical polymerizable formulation are selected from the group consisting of ascorbic acid, tartaric acid, or other hydroxy-carboxylic acids.

In an embodiment of the present disclosure, there is provided an ingestible device for collecting samples from a subject as described herein, wherein the chemical polymerizable formulation swells between 1X to 10000X times the original volume of the polymerizable formulation.

In an embodiment of the present disclosure, there is provided an ingestible device for collecting samples from a subject as described herein, wherein the device is ingestible and wherein the sampling by the device is non-invasive.

In an embodiment of the present disclosure, there is provided an ingestible device for collecting samples from a subject as described herein, wherein the device is a micromechanical device.

The encapsulated material is suitable for downstream analysis, including DNA/RNA extraction, microbial profiling (e.g., 16S rRNA, ITS sequencing), proteomics, or metabolomics, among others using a variety of high-throughput platforms including, but not limited to, nanopore sequencing, Illumina systems, and emerging multiplexed genomic techniques.

In an embodiment of the present disclosure, there is provided an ingestible device for collecting samples from a subject, the device comprising:
- a core shell (2) having a plurality of outer wall defining an inner volume;
- a separator passage (6) disposed in the inner volume and adapted to define the inner volume of core shell as a sample chamber (4);
- an inlet passage (8) in fluid communication with the sample chamber (4) of the core shell;
- an activation mechanism (12) disposed in the sample chamber (4) and is adapted to allow ingress of sample from the inlet passage (8) to the sample chamber (4) though the separator passage (6).

In an embodiment of the present disclosure, there is provided an ingestible device for collecting samples from a subject as described herein, wherein the activation mechanism (12) is a mechanical based activation mechanism, preferably a spring-based-bead closing mechanism.

In an embodiment of the present disclosure, there is provided an ingestible device for collecting samples from a subject as described herein, wherein the spring-based-bead closing mechanism (12) comprises an actuator (13) positioned at the solid base of the core shell (2) and is configured to advance the supporter plate (11) towards the separator passage (6) upon dissolution of dissolvable blocks (12).

In an embodiment of the present disclosure, there is provided an ingestible device for collecting samples from a subject, the device comprising:
- a core shell (2) having one end opening and a solid base;
- a separator passage (6) disposed at one end opening of the core shell (2) and is adapted to define the inner volume of core shell as a sample chamber (4);
- an inlet passage (8) in fluid communication with the sample chamber (4) of the core shell;
- a plurality of dissolvable blocks (12) mounted on the surface of supporter plate (11) and is configured to restrict the movement of supporter plate before sample collection;
- an actuator (13) positioned at the solid base of the core shell (2) and is configured to advance the supporter plate towards the separator passage (6) upon dissolution of dissolvable blocks (12).

In an embodiment of the present disclosure, there is provided an ingestible device for collecting samples from a subject as described herein, wherein the dissolvable blocks (12) comprises GRAS, non-toxic chemical species, including but not limited to effervescent compositions as such or tableted forms thereof as those formed from solid carbonates (e.g., sodium bicarbonate) and solid acids (e.g., acetylsalicylic acid, citric or tartaric acid), optionally embedded in a water-soluble polymer matrix; sugar-based blocks (e.g., mannitol, sorbitol, xylitol); or salt-based blocks. These compositions may incorporate binders, humectants (e.g., zeolite), or starch derivatives to impart mechanical stability and microbial composition preserving properties, enabling controlled dissolution upon contact with the luminal fluids in the GI tract.

In an embodiment of the present disclosure, there is provided an ingestible device for collecting samples from a subject as described herein, wherein the actuator is a spring.

In an embodiment of the present disclosure, there is provided an ingestible device for collecting samples from a subject, the device comprising:
- a core shell (2) having a plurality of outer wall defining an inner volume;
- a separator passage (6) disposed in the inner volume of the core shell (2) and is adapted to define the inner volume of core shell as a sample chamber (4);
- an inlet passage (8) in fluid communication with the sample chamber (4) of the core shell;
- an activation mechanism (5) disposed in the sample chamber (4) and is adapted to allow ingress of sample from the inlet passage to the sample chamber (4) through the separator passage (6).

In an embodiment of the present disclosure, there is provided an ingestible device for collecting samples from a subject as described herein, wherein the core shell is a cylindrical core shell.

In an embodiment of the present disclosure, there is provided an ingestible device for collecting samples from a subject as described herein, wherein the device core shell (2) with maximum length ranges from 5 mm to 25 mm is for small animal study.

In an embodiment of the present disclosure, there is provided an ingestible device for collecting samples from a subject as described herein, wherein the device core shell (2) with external diameter ranges from 1 mm to 5 mm is for small animal study.

In an embodiment of the present disclosure, there is provided an ingestible device for collecting samples from a subject as described herein, wherein the device core shell (2) with maximum length ranges from 10 mm to 30 mm is for large animal and human study.

In an embodiment of the present disclosure, there is provided an ingestible device for collecting samples from a subject as described herein, wherein the device core shell (2) with external diameter ranges from ranging from 3 mm to 15 mm is for large animal and human study.

In an embodiment of the present disclosure, there is provided an ingestible device for collecting samples from a subject as described herein, wherein the inlet passage (8) allows flow of intestinal fluid to enter in activation chamber (3) and sample chamber (4).

In an embodiment of the present disclosure, there is provided an ingestible device for collecting samples from a subject as described herein, wherein the shape of inlet passage (8) is selected from the group consisting of circular, elliptical and polygonal.

In an embodiment of the present disclosure, there is provided an ingestible device for collecting samples from a subject as described herein, wherein the inlet passage (8) of width in the range of 0.5 mm to 5 mm is for small animal study.

In an embodiment of the present disclosure, there is provided an ingestible device for collecting samples from a subject as described herein, wherein the inlet passage (8) of width in the range of 1 mm to 20 mm is for human or large animal study.

In an embodiment of the present disclosure, there is provided an ingestible device for collecting samples from a subject as described herein, wherein the inlet passage (8) of length in the range of 0.5 mm to 25 mm is for small animal study.

In an embodiment of the present disclosure, there is provided an ingestible device for collecting samples from a subject as described herein, wherein inlet passage (8) of length in the range of 1 mm to 30 mm is for human or large animal study.

In an embodiment of the present disclosure, there is provided an ingestible device for collecting samples from a subject as described herein, wherein the device core shell (2) comprises an activation chamber (3) comprising chemical activation via a polymer swelling and/or polymer formation mechanism (e.g., via a hydrogel or a pre polymer mix,) mechanical or electrical actuation system to close the device core shell (2) and prevent cross contamination.

In an embodiment of the present disclosure, there is provided an ingestible device for collecting samples from a subject as described herein, wherein the length of the activation chamber (3) in the range of 0.5 mm to 25 mm is for small animal study.

In an embodiment of the present disclosure, there is provided an ingestible device for collecting samples from a subject as described herein, wherein the length of the activation chamber (3) in the range of 1 mm to 30 mm is for large animal or human study.

In an embodiment of the present disclosure, there is provided an ingestible device for collecting samples from a subject as described herein, wherein the activation chamber (3) is separated from the sample chamber (4) by a separator passage (6).

In an embodiment of the present disclosure, there is provided an ingestible device for collecting samples from a subject as described herein, wherein the separator passage (6) allows the intestinal fluid to come inside the sample chamber (4) from the activation chamber (3).

In an embodiment of the present disclosure, there is provided an ingestible device for collecting samples from a subject as described herein, wherein the separator passage (6) is of shape selected from the group consisting of circular, elliptical and polygonal shape.

In an embodiment of the present disclosure, there is provided an ingestible device for collecting samples from a subject as described herein, wherein the length of separator passage (6) in the range of 0.5 mm to 25 mm is for small animal study.

In an embodiment of the present disclosure, there is provided an ingestible device for collecting samples from a subject as described herein, wherein the length of separator passage (6) in the range of 1 mm to 30 mm is for human or large animal study.

In an embodiment of the present disclosure, there is provided an ingestible device for collecting samples from a subject as described herein, wherein the width of separator passage (6) in the range of 0.5 mm to 5 mm is for small animal study.

In an embodiment of the present disclosure, there is provided an ingestible device for collecting samples from a subject as described herein, wherein the width of separator passage (6) in the range of 1 mm to 30 mm is for human or large animal study.

In an embodiment of the present disclosure, there is provided an ingestible device for collecting samples from a subject as described herein, wherein the wall of the separator passage (6) is selected from the group consisting of circular, elliptical and polygonal shape.

In an embodiment of the present disclosure, there is provided an ingestible device for collecting samples from a subject as described herein, wherein the wall of the separator passage (6) is of curvature geometries selected from the group consisting of semi-circular, elliptical, parabolic, hyperbolic, cubic, concave, convex, and flat.

In an embodiment of the present disclosure, there is provided an ingestible device for collecting samples from a subject as described herein, wherein the device core shell (2) comprises a separate sample chamber (4) to protect the sample from cross contamination.

In an embodiment of the present disclosure, there is provided an ingestible device for collecting samples from a subject as described herein, wherein the sample chamber of the device of length in the range of 5 mm to 25 mm is for small animal study.

In an embodiment of the present disclosure, there is provided an ingestible device for collecting samples from a subject as described herein, wherein the sample chamber of the device of length in the range of 10 mm to 30 mm is for human or large animal study.

In an embodiment of the present disclosure, there is provided an ingestible device for collecting samples from a subject as described herein, wherein the diameter of the sample chamber in the range of 1 mm to 5 mm is for small animal study.

In an embodiment of the present disclosure, there is provided an ingestible device for collecting samples from a subject as described herein, wherein the diameter of the sample chamber in the range of 3 mm to 10 mm is for human or large animal study.

In an embodiment of the present disclosure, there is provided an ingestible device for collecting samples from a subject as described herein, wherein the core shell (2) of the device comprises a top cover (7) configured to cover only the sample chamber (4) to protect samples from contamination from above.

In an embodiment of the present disclosure, there is provided an ingestible device for collecting samples from a subject as described herein, wherein the core shell (2) of the device comprises a top cover (7) configured to cover both activation chamber (3) and sample chamber (4) hence protecting them from external environments and also improve the streamline flow of the device.

In an embodiment of the present disclosure, there is provided an ingestible device for collecting samples from a subject as described herein, wherein the core shell (2) of the device is of thickness in the range of 0.1 mm to 5 mm.

In an embodiment of the present disclosure, there is provided an ingestible device for collecting samples from a subject as described herein, wherein the device is fabricated using high-resolution additive manufacturing, including but not limited to stereolithography (SLA), two-photon polymerization, or any method capable of achieving dimensional tolerances ≤25 µm in X, Y, and Z axes. These tolerances are functionally required for integration of micro actuation chambers, fluidic inlets, and sample encapsulation compartments necessary for site-specific gastrointestinal sampling and safe egestion. These device size range in any dimensional tolerance.

In an embodiment of the present disclosure, there is provided an ingestible device for collecting samples from a subject as described herein, wherein the device is optionally tethered with an ultra-thin biocompatible retrieval string that can be easily removed from the body post-sampling.

In an embodiment of the present disclosure, there is provided an ingestible device for collecting samples from a subject as described herein, wherein the device is configured to securely store sampled biomolecules inside the device with minimal cross contamination until its natural egestion or gastric emptying, after which the sampled material can be subjected to further bio/chemical treatment including but not limited to genomics, proteomics, metabolomics, and a combination thereof.

In an embodiment of the present disclosure, there is provided an ingestible device for collecting samples from a subject as described herein, wherein the device includes an internal stabilization mechanism to preserve collected biomolecules and microbiota at their original concentrations by maintaining a controlled environment within the collection chamber, preventing degradation until egestion.

In an embodiment of the present disclosure, there is provided an ingestible device for collecting samples from a subject as described herein, wherein the device includes microfluidic or nanofluidic channels to direct samples to plurality of compartments with specific preservation agents, such as enzymatic inhibitors, ensuring biomolecule integrity for subsequent analyses.

In another aspect of the present disclosure, there is provided a process of collecting a gastrointestinal sample by the ingestible device as described herein.

In an embodiment of the present disclosure, there is provided a process of collecting a gastrointestinal sample by the ingestible device as described herein, the process comprising:
(a) actuating the device for sample collection from a specific site of the gastrointestinal tract;
(b) collecting sample from the specific site of the gastrointestinal tract with precise spatial resolution along the gastrointestinal (GI) tract;
(c) storing the sample from the specific site of the gastrointestinal tract inside the ingestible device; and
(d) repeating the steps (a) to (c) at different sites along the gastrointestinal tract.

In an embodiment of the present disclosure, there is provided a process of collecting a gastrointestinal sample as described herein, wherein the process further comprises tracking the device through a magnetic or radio-frequency identification (RFID)-based retrieval system configured for locating real-time tracking the device within the GI tract for site-specific sampling.

In an embodiment of the present disclosure, there is provided a process of collecting a gastrointestinal sample as described herein, wherein the sample includes luminal fluids, cells, nucleic acids and ribonucleic acids, enzymes, proteins, and microbiome, and combination thereof.

In an embodiment of the present disclosure, there is provided a process of collecting a gastrointestinal sample as described herein, wherein the sample is collected along the entire length of the gastrointestinal tract, including specific sites such as the stomach, pylorus, duodenal region, and intestinal lumen including ileum, jejunum, large intestine, and colon.

In an embodiment of the present disclosure, there is provided a process of collecting a gastrointestinal sample as described herein, wherein the sample is collected into microfluidic or nanofluidic channels to direct samples to compartments of the devices with specific preservation agents, such as enzymatic inhibitors, ensuring biomolecule integrity for subsequent analyses.

In an embodiment of the present disclosure, there is provided a process of collecting a gastrointestinal sample as described herein, wherein the process includes stabilizing the preserves collected biomolecules and microbiota at their original concentrations by maintaining a controlled environment within the collection chamber/compartments, preventing degradation until egestion.

Although the subject matter has been described in considerable detail with reference to certain preferred embodiments thereof, other embodiments are possible.

### EXAMPLES

The disclosure will now be illustrated with working examples, which is intended to illustrate the working of disclosure and not intended to take restrictively to imply any limitations on the scope of the present disclosure. Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods, the exemplary methods, devices and materials are described herein. It is to be understood that this disclosure is not limited to particular methods, and experimental conditions described, as such methods and conditions may vary.

### EXAMPLE 1: Fabrication of the Ingestible Microdevices

Devices were fabricated using Stereolithography (SLA)-based 3D printing. Feature-based CAD modelling was performed in SolidWorks (Dassault Systems), followed by tessellation into STL format for slicing. Slicing, support structures, and raft generation were carried out in Formlabs^{®} PreForm software to prevent peeling forces and avoid cup formation. A slice thickness of 25 µm was set for high-precision printing.

The Formlabs^{®} 3B+ SLA printer, equipped with an 85 µm laser (405 nm, 250 mW) and a resolution of 20 µm (X/Y) and 25 µm (Z), was used. Formlabs^{®} ClearV4 resin was employed as the negative photoresist. Printed devices were removed and washed in 99% IPA using Form Wash (15 min, spin), dried at room temperature (30 min), and subjected to a second wash (15 min). Post-wash, support structures were removed, and devices were cured in Form Cure (405 nm UV, 35°C, 30 min) to eliminate residual uncured resin.

While SLA-based printing with Formlabs^{®} 3B+ and ClearV4 resin is used in prototyping, the present disclsoure encompasses any fabrication route-additive or subtractive-that preserves the structural, mechanical, and biochemical compatibility required for the claimed sampling, sealing, and fecal-egestion functions. Suitable materials encompass a broad range of biocompatible, non-biodegradable soft polymers and elastomers, including medical-grade silicones, polyurethanes, polyether block amides, thermoplastic elastomers, styrene-based copolymers, fluoropolymers, polyethylene, polypropylene, polycarbonate, polysulfone, polyethersulfone, and polymethyl methacrylate, among others. Metallic, non-metallic, and ceramic materials such as stainless steel, titanium, nitinol, glassy carbon, and zirconia may also be employed depending on design requirements.

### EXAMPLE 2: Loading of Devices with pre-polymer mixture

The pre-polymer mixture comprises of biocompatible acids like ascorbic acid, citric acid or tartaric acid, among others coupled with FeCl₃ (iron (III) chloride, iron trichloride), and Ammonium Persulfate (APS) serving as redox radical initiators. A modified spatula loading method was used to load the resulting reactant mixture into the devices. Then the inlet port was closed via a polymer swelling mechanism where the hydrogel swells several times its original volume. This swelling ratio could be anywhere between 1X to 10000X times the original volume of the pre-polymer mix. The resulting hydrogel could further serve the function of closing the inlet port, enmeshing the sampled luminal fluids within the hydrogel matrix, and creating entry barrier between one of more inner chambers.

### EXAMPLE 3: PEGDA hydrogel formation and swelling

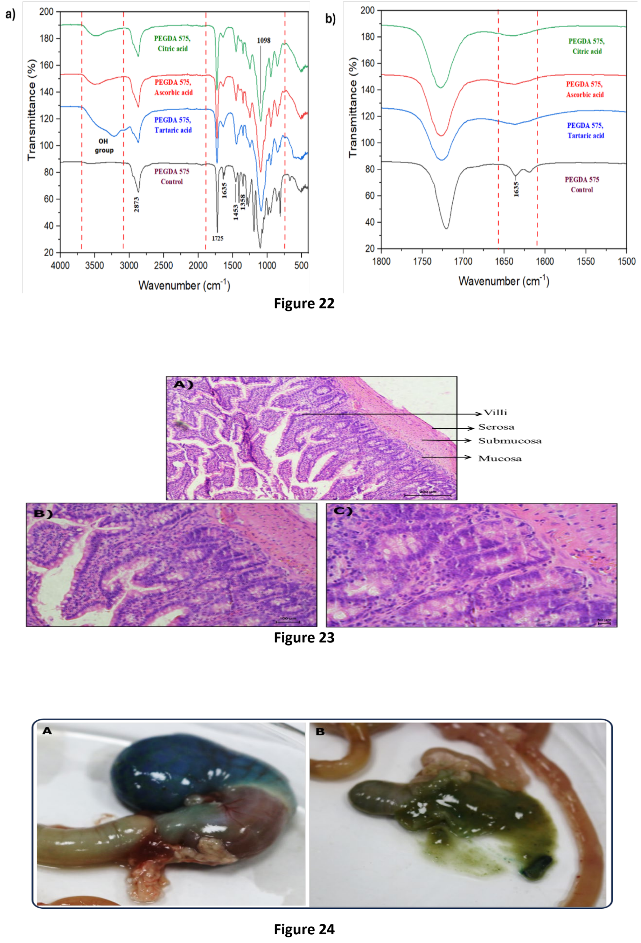

Polymerisation of PEGDA monomer (m.w. 575 and 700) in biocompatible redox initiation systems and subsequent vinyl group polymerization was assessed via ATR-FTIR spectroscopy, as shown in Figure 22 of the present disclosure. Synthesis of the targeted-radical polymerization mechanism of the vinyl-group monomers (PEGDA m.w. 575, Radical initiators as FeCl₃-APS, and biocompatible acids. The biocompatible acids include but not limited to ascorbic acid; tartaric acid; and citric acid. In another embodiment, the choice of biocompatible acid as a redox agent could have pKa₁ values in the range of 2.5-5.

FTIR characterization of the resulting PEGDA polymers C-H stretching (from the acrylate groups): ~2850-2950 cm⁻¹, O-H stretching: Broad band around 3200-3600 cm⁻¹, C-O-C stretching (ether bond): ~1098 cm⁻¹, C=O stretching: ~1700-1750 cm⁻¹, Alkene Bending: 1635 cm⁻¹, Bending, CH2 and CH3 deformation 1453 cm⁻¹, C-H (Bending) : 1440-1470 cm⁻¹. C) The peak at 1635 cm-1 assigned to the C=C in PEGDA 575 structure, which disappears during the radical polymerization via all three acids viz a viz ascorbic acid, citric acid and tartaric acid based confirming vinyl-group polymerization.

### EXAMPLE 4: Oral gavaging and histological assessment confirming device safety

Adult male Sprague-Dawley rats (190-250 g) were housed in shoebox cages under controlled humidity, a 23±2°C temperature, and a 12-hour light/dark cycle. They received standard laboratory chow and water. After one week of acclimatization, devices were orally administered via. gavage following designated protocols. Device embodiments were administered to assess sampling inside the GI tract and device egestion due to peristalsis. After the device administration, animals were sacrificed, and devices with tissue samples were collected and stored at -80°C. A 1-2 cm² tissue section was preserved in 4% paraformaldehyde, while the rest was frozen at -20°C.

Since the aim of the present disclosure was to verify devices activation or actuation in vivo resulting in sampling of the GI luminal content, and subsequent entrapment of biomolecules, like gut microbiota, the excised animal tissue and fecal sample from served as a control. After euthanasia, the specific sections of the GI tract were removed from the rats that had been administered devices in order to localize the position of the devices (stored at -80 °C). Histological assessment confirmed shows a cross-sectional area of the excised small intestine tissue (jejunum) with unaffected mucosa characterized by slender villi and uniform crypts, showing no signs of cellular inflammatory response, as shown in Figure 23 of the present disclosure.

### EXAMPLE 5: Ingestible Microdevice for Nucleic acids and Proteins isolation and analyses.

Microdevices were retrieved from distinct anatomical regions of the gastrointestinal (GI) tract-including but not limited to the stomach, jejunum, duodenum, proximal and distal ileum, cecum, and colon-following oral administration in a controlled in vivo study. Additional retrieval occurred via fecal excretion. For comparative analysis, matched surrounding tissue samples from device-adjacent GI sites, as well as fecal material (~0.5-1 g), were collected as control inputs.

To extract microbial genomic material, a standardized lysis and purification workflow was employed, adapted to suit the unique composition and polymeric housing of the microdevices. Devices were physically disrupted prior to lysis, while control samples (tissue and feces) were processed directly. Thermal and mechanical disruption protocols were utilized to enhance nucleic acid recovery, incorporating elevated lysis temperatures (e.g., ≥60°C) and agitation (e.g., highspeed mixing or vortexing) for an extended duration (e.g., ≥15 min).

Post-lysis, column-based purification was employed with modifications to elution temperature and buffer volumes to maximize yield and recovery. Total elution volumes were adjusted to ≤100 µL, enabling concentration of low-abundance genomic templates from in vivo environments.

Amplification of target microbial genes was performed via a dual-stage (e.g., semi-nested) PCR approach. In the initial amplification, conserved bacterial 16S rRNA regions were targeted using universal primers (e.g., 27F and 1492R or equivalents). Resultant amplicons were used for library construction employing a native barcode adapter system optimized for multiplexed, long-read sequencing platforms. This integrated approach enabled high-resolution taxonomic profiling from spatially localized GI samples, as validated by DNA yield metrics and downstream sequencing quality parameters, as shown in Table 1 below:

**Table 1: Concentration readings of semi-nested PCR used for products End prep using Qubit 4 fluorometer.**

| Stage | Sample type | Concentration (ng/µL) | Quantity (ng) |
|---|---|---|---|
| PCR and post-PCR End prep | Device | 6.85 ± 4.578 | 54.8 ± 36.623 |
| | Tissue | 4.147 ± 3.367 | 33.178 ± 26.935 |
| | Faces | 13.556 ± 4.493 | 108.448 ± 35.945 |
| Barcoding with oligonucleotides | Device | 6.85 ± 4.578 | 25.688 ± 17.167 |
| | Tissue | 4.147 ± 3.367 | 15.552 ± 12.626 |
| | Faces | 13.556 ± 4.493 | 50.835 ± 16.849 |
| Adapter ligation | | 2.32 | 69.6 |
| Loaded library | | 1.12 | 13.44 |

Alkaline phosphatase (ALP) concentration was measured using a colorimetric enzymatic assay. In this method, ALP catalyzes the hydrolysis of benzene disodium phosphate to generate free phenol and phosphoric acid. The liberated phenol subsequently reacts with 4-aminopyrine under alkaline conditions and is oxidized by potassium ferricyanide to form a red quinoneimine dye. The enzyme activity is indirectly quantified by measuring the optical density (OD) at 520 nm using a microplate reader. The assay was performed using the Elabscience^{®} ALP Enzyme Assay Kit, strictly following the manufacturer's instructions. A standard calibration curve was generated (R² = 0.9995), and the ALP enzyme concentration in the sample was determined to be 0.00656 ± 0.00092 mg/mL.

### EXAMPLE 6: Site-specific GI anatomical marking and Sampling via Dyes & Fiducial agents.

An ingestible microdevice with a dual-chamber design was administered to a murine model. The lower chamber contained a redox-polymerizable PEGDA formulation, and the upper chamber held methylene blue dye. An enteric coating delayed activation until intestinal pH was encountered. Upon coating degradation, methylene blue was released to visually mark the activation site, as shown in Figure 24 of the present disclosure. Concurrently, luminal fluid triggered in-situ polymerization, entrapping the site-specific microbiota.

### EXAMPLE 7: Genomic sequencing of the isolated gut microbiome from the Microdevice egested via the fecal route.

The method comprises: (a) extracting the sample directly from the isolated internal compartment of the egested microdevice; (b) preparing sequencing libraries using an optimized version of the Native Barcoding Kit 24 V14 (SQK-NBD114.24), with specific modifications to accommodate the molecular characteristics and low-nucleic-acid yield of microdevice-derived GI luminal fluid; (c) performing end-prep with precise volumetric adjustments (e.g., 8 µl sample + 3.5 µl Nuclease Free Water + 1 µl DNA Control Sample + 1.75 µl Reaction Buffer + 0.75 µl Enzyme Mix), thermal incubation at 20°C and 65°C, barcode ligation under ice conditions, reaction termination with EDTA, pooling, bead-based clean-up using 0.4x AMPure XP Beads, elution using Nuclease-Free Water, and downstream adapter ligation and flow cell loading, wherein the entire protocol is configured to maintain sample integrity derived from the microdevice's protective compartment.

The present disclosure describes a method for downstream analysis of genomic sequencing data derived from gastrointestinal luminal fluid samples collected via the ingestible microdevice described herein. Following sample retrieval post-egestion, library preparation is performed using the SQK-NBD114.24 Native Barcoding Kit with optimized modifications to the manufacturer's protocol, including controlled dilution of low-input DNA samples and inclusion of DNA control spike-ins, followed by thermal incubation at 20°C for 5 minutes and 65°C for 5 minutes, barcode ligation on ice, bead-based purification using 0.4× AMPure XP beads, elution, and Qubit 4 quantification. Final libraries are loaded onto an FLO-MIN114 nanopore flow cell for sequencing. The method comprises:
a) base calling raw POD5 files generated by the FLO-MIN114 sequencing flow cell using the Dorado base calling framework (version 7.8.3), specifically employing the Super-Accurate model configured for 400 base pairs per second (bps), wherein reads are filtered using a minimum quality score threshold of Q≥9 to ensure high-fidelity sequence data;
b) performing taxonomic classification of the resulting FASTQ reads using the minimap2-based classification algorithm (version 2.28-r1209), aligned against a curated reference database NCBI_16S_18S ribosomal RNA sequences.
c) importing the resulting taxonomic abundance tables into the R statistical environment and executing downstream statistical and visual analyses using the phyloseq and ggplot2 packages, wherein microbial community composition, alpha-diversity, and beta-diversity metrics are computed and visualized for interpretation of site-specific gastrointestinal microbial profiles. The results are shown in Table 2 below:

**Table 2: Relative abundance (%) of the top seven microbial species detected in samples from the ingestible microdevice, the duodenal site of device activation, and the co-egested fecal sample following non-invasive recovery.**

| **Top-seven species** | **Egested Microdevice via fecal route** | **Site of device activation in the GI tract: Control Tissue (duodenum)** | **Fecal sample co-egested alongside the microdevice** |
|---|---|---|---|
| *Romboutsia timonensis* | 0.335 | 0.3132 | 0.5022 |
| *Staphylococcus arlettae* | 0.1018 | 0.0680 | 0.0041 |
| *Corynebacterium stationis* | 0.0425 | 0.0047 | 0.0011 |
| *Staphylococcus ureilyticus* | 0.0375 | 0.0785 | 0.0062 |
| *Blautia glucerasea* | 0.0375 | 0.0063 | 0.0001 |
| *Clostridium saudiense* | 0.035 | 0.0168 | 0.0733 |
| *Lactobacillus gallinarum* | 0.005 | 0.0042 | 0.0893 |

The present disclosure provides an ingestible microdevice system for site-specific gastrointestinal sampling with integrated containment and actuation mechanisms enabling safe fecal egestion. The device ensures high-integrity capture of luminal contents-including biomolecules, microbiota, and biochemical markers-not replicable by conventional methods. Coupled with a defined downstream genomic and metagenomic analysis pipeline, it enables robust, reproducible, and clinically relevant profiling.

## Claims

1. An ingestible device for collecting samples from a subject, the device comprising:
- a core shell (2) having a plurality of outer wall defining an inner volume;
- a separate passage (6) disposed in the inner volume and adapted to divide the inner volume into one or more chambers selected from activation chamber (3) and sample chamber (4);
- an inlet passage (8) in fluid communication with the chamber selected from activation chamber (3) and sample chamber (4) of the core shell; and
- an activation mechanism (5,12) disposed in the chamber selected from activation chamber (3) and sample chamber (4) and is adapted to allow ingress of sample from the inlet passage (8) to the sample chamber (4) though the separator passage (6).

2. The device as claimed in claim 1, wherein the device comprises a pH sensitive protective outer shell (1) mounted partially on the outer surface of the core shell (2), and wherein the device comprises a top cover (7) disposed at a mouth of the core shell and covered by the pH sensitive protective outer shell (1), and wherein the device comprises a detectable marking agent chamber (3) disposed in the inner volume of core shell and is covered partially by the pH sensitive protective outer shell (1).

3. The device as claimed in claim 1, wherein the activation mechanism is selected from the group consisting of chemical, electrical, and mechanical based system, and wherein the activation mechanism is adapted to close the sample chamber upon the lapse of a predefined period of time from a first instance of collection of sample in the sample chamber.

4. The device as claimed in claim 3, wherein the chemical-based activation mechanism is a chemical polymerizable formulation, and wherein the chemical polymerizable formulation comprises at least one polymerizable monomer or hydrogel prepolymer and a redox/radical initiator.

5. The device as claimed in claim 4, wherein the polymerizable monomer or hydrogel prepolymer comprises a moiety selected from the group consisting of carboxylic acids, carboxylic acid anhydrides, sulfonic acids, dimethyl acrylamide, diacetone acrylamide, hydroxyl ethyl methacrylate, hydroxyl ethyl acrylate, dimethylaminoethyl acrylate, dimethylaminoethyl methacrylate, ethoxy ethoxy ethyl methacrylate, ethoxy ethyl methacrylate, methyl acrylate, poly (ethylene glycol) diacrylate (PEGDA), ethyl acrylate, propyl acrylate, butyl acrylate, methyl methacrylate, acrylic acid, methacrylic acid, maleic acid, cinnamic acid, itaconic acid, crotonic acid, ethacrylic acid, citoconic acid, mesaconic acid, fumaric acid, β-sterylacrylic acid, acrylate esters, acrylamides, olefins, vinyl esters, vinyl ethers, vinyl amides, 2-acrylamido-2-methylpropane sulfonic acid (AMPS), and 3-sulphopropyl acrylate (SPA), and wherein the redox/radical initiator system is selected from transition metal salts such as iron (III) chloride, peroxides, persulfates, and organic reducing agents; and wherein the organic reducing agents are selected from the group consisting of ascorbic acid, tartaric acid, or other hydroxy-carboxylic acids.

6. The device as claimed in claim 4, wherein the chemical polymerizable formulation swells between 1X to 10000X times the original volume of the polymerizable formulation.

7. The device as claimed in claim 2, wherein the detectable marking agent is selected from the group consisting of dyes, pigments, fiducial markers, radiopaque agents, nanoparticles, enzymes coated particles, chromophores and fluorophores.

8. The device as claimed in claim 2, wherein the pH sensitive protective outer shell (1) is selected from enteric coating and a combination of enteric coating and animal/plant-based gelatine with additives.

9. The device as claimed in claim 3, wherein the mechanical-based activation mechanism is a spring-based-bead closing mechanism, and wherein the spring-based-bead closing mechanism comprises an actuator (13) positioned at the solid base of the core shell (2) and is configured to advance the supporter plate (11) towards the separate passage (6) upon dissolution of dissolvable blocks (12).

10. A process for collecting a gastrointestinal (GI) sample using the ingestible device of claim 1, the process comprising:
a) actuating the device for sample collection from a specific site of the gastrointestinal tract;
b) collecting sample from the specific site of the gastrointestinal tract with precise spatial resolution along the gastrointestinal tract;
c) storing the sample collected from the specific site of the gastrointestinal tract inside the ingestible device;
d) repeating the steps (a) to (c) at different sites along the gastrointestinal tract; and
e) optionally comprising tracking the device through a magnetic or radio-frequency identification (RFID)-based retrieval system configured for locating real-time tracking the device within the GI tract for site-specific sampling.
